Europäisches Patentamt

**European Patent Office**

**Office européen des brevets**

(11) Publication number: **0 411 187 A1**

# EUROPEAN PATENT APPLICATION

(21) Application number: **89114360.4**

(22) Date of filing: **03.08.89**

(51) Int. Cl.⁵: **A61L 2/10, A47J 47/00**

(43) Date of publication of application:
**06.02.91 Bulletin 91/06**

(84) Designated Contracting States:
**AT BE CH DE ES FR GB GR IT LI LU NL SE**

(71) Applicant: **Liaw, Min-Jenn**
**9th Floor No.1291, Chern Der Road**
**Taipei City(TW)**

(72) Inventor: **Liaw, Min-Jenn**
**9th Floor No.1291, Chern Der Road**
**Taipei City(TW)**

(74) Representative: **Brown, John David et al**
**FORRESTER & BOEHMERT**
**Widenmayerstrasse 4/I4/I**
**D-8000 München 22(DE)**

(54) **A container for storing and sterilizing a chopping board.**

(57) The present invention relates to a container for storing and sterilizing a chopping board which mainly comprises a case (60), a scattering plate (61), a sterilizing lamp (62), a screen cover (63), a water collecting box (65), a supporting plate (66), a microswitch (67), a compression spring (68), a holding plate (69), a heating element (70) and a bottom plate (71). The weight of the chopping board forces the supporting plate (66) to contact the microswitch (67) which in turn actuates both the sterilizing lamp (62), and the heating element (70), such that the ultraviolet light generated by the lamp (62) together with the heat produced by the heating element (70) can be scattered by the scattering plate (61) to improve both sterilizing and drying efficacy. The present container has a compact overall volume and can be easily assembled from a few simple components.

FIG. 1

## A CONTAINER FOR STORING AND STERILIZING A CHOPPING BOARD

The present invention relates to a container and particularly to one which is used to store and sterilize a chopping board.

The sanitary requirements for the appliance to be used in the kitchen are increasing day by day. Although many newly designed devices, such as rice container, dish dryer, etc. have already been provided for such purposes, to date we still lack a proper container to store and to sterilize the chopping board which, otherwise, may easily be polluted.

It is, therefore, an object of the present invention to obviate and mitigate the above-identified deficiency.

It is an object of the present invention to provide a container for storing and sterilizing a chopping board which has a simplified structure with a compact volume and is easy to be assembled.

It is another object of the present invention to provide a container for storing and sterilizing a chopping board which comprises a scattering plate by which the ultraviolet light generated by a sterilizing lamp as well as the heat produced by a heating element can be uniformly scattered so as to improve both sterilizing and drying efficacy.

It is another object of the present invention to provide a container for storing and sterilizing a chopping board which can uniformly distribute the heat produced by the heating element to provide a better drying effect.

In order that the invention may be readily understood, embodiments thereof will now be described, by way of example, with reference to the accompanying drawings, in which:

FIG. 1 is a fragmentary view of a first preferred embodiment of the present invention,

FIG. 2 is a cut-away view of the first preferred embodiment as shown in FIF. 1;

FIG. 3 is a perspective view of a second preferred embodiment of the present invention;

FIG. 4 is a perspective view of a third preferred embodiment of the present invention;

FIG. 5 is a perspective view of a fourth preferred embodiment of the present invention;

FIG. 6 is a fragmentary view of a fifth preferred embodiment of the present invention; and

FIG. 7 is a cut-away view of the fifth preferred embodiment as shown in FIG. 6.

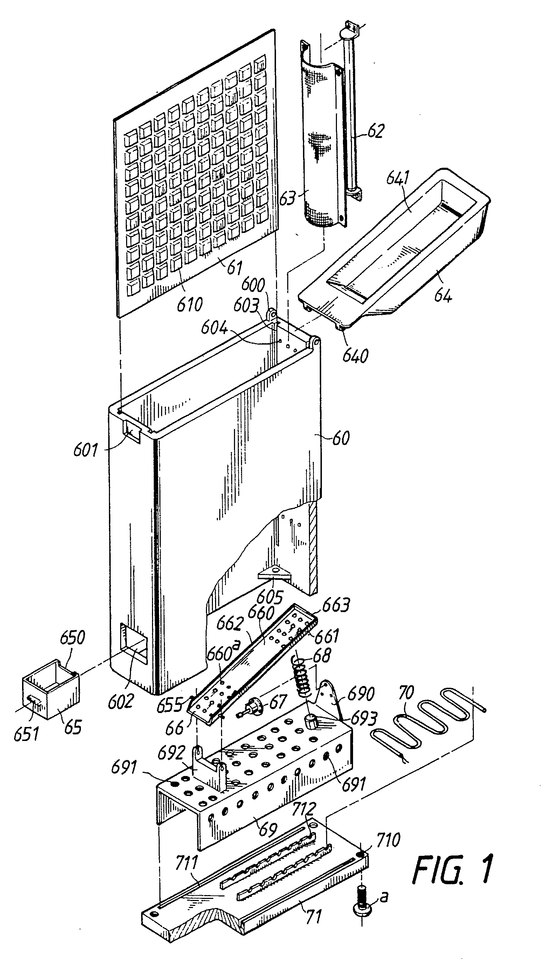

Referring to FIG. 1, a first preferred embodiment of the present invention mainly comprises a rectangular hollow case 60, one or more scattering plates 61, one or more sterilizing lamps 62, a screen cover 63, an upper cover 64, a water collecting box 65, a supporting plate 66, a microswitch 67, a compression spring 68, a holding plate 69, a heating element 70 and a bottom plate 71. The rectangular hollow case 60 is formed at the upper portion of one of its longitudinal side walls with a pair of ears 600 to which the upper cover 64 is hinged and at the other longitudinal side wall with a recessed portion 601 to facilitate the opening of the upper cover 64. The case 60 further is provided at the bottom with an opening 602 through which the water collecting box 65 can pass to be disposed in the case 60. The inner walls of the case 60 are furnished with slots 603 for engaging with the scattering plates 61 and with a plurality threaded holes 604 for locating the sterilizing lamps 62 and the screen cover 63. The four lower corners of the case 60 are properly formed with four fixing pieces 605 by which the bottom plate 71 is threadedly secured to the case 60. The scattering plate 61 is made by punching its smooth surface to form a plurality of projecting portions 610 in order to serve as a reflective plate. The upper cover 64 is provided with a pair of flanges 640 corresponding to the ears 600 of the case 60, and with a centrally located recess 641. The water collecting box 65 has a recessed portion 650 through which the open end of the supporting plate 66 passes, and a knob 651 by which the water collecting box 65 can be easily drawn out of the case 60. The supporting plate 66 comprises a plate surface 660 having a plurality of protuberances 600a, three side walls 661, 662 and 663 respectively surrounding its three side and projecting upward from the plate surface, a projecting rod 664 formed on the underside of the plate surface 660 to be engaged with one end of the compression spring 68, and a pair of pins 665 disposed at two longitudinal sides 661 and 662 thereof. The holding plate 69 has an inverted U-shaped body having a plurality of holes 691 and is formed with a connection member 690 to which the microswitch 67 is secured. The upper surface of the holding plate 69 is properly formed with a fixing seat 692 by which the supporting plate 66 is supported and an inclined stud 693 for engaging with the other end of the compression spring 68. The heating element 70 is an S-shaped or the like heating tube. The bottom plate 71 is designed to be disposed in the hollow interior of the case 60. The four corners of the bottom plate 71 have four through holes 710. The bottom plate 71 is provided along its two sides with a pair of grooves 711 with which the holding plate 69 is engaged, and at the center with two parallel rails 712 on which the heating element 70 is located.

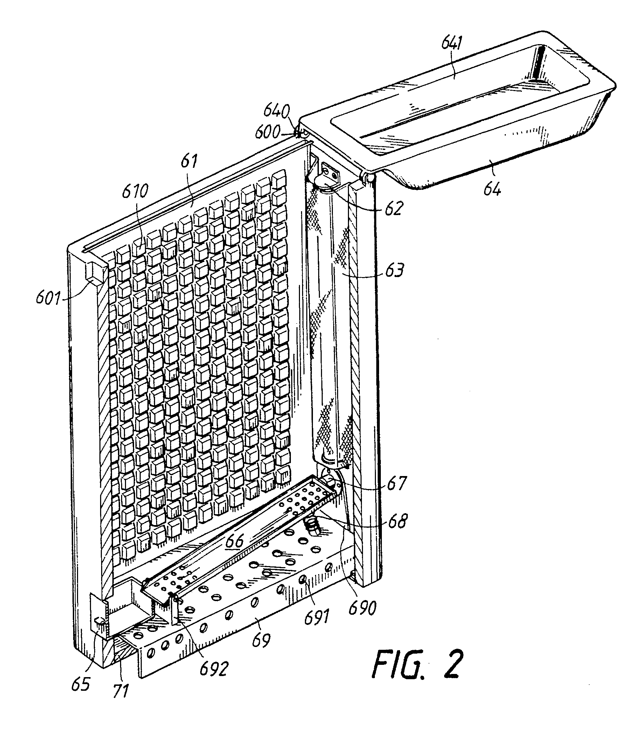

Referring to FIG. 2, in assembly, the sterilizing lamp 62 together with the screen cover 63 are secured to the threaded holes 604 of the case 60 by means of serews or the like. Then, the microswitch 67 is fixed to the connection member 690 of the holding plate 69. One end of the compression spring 68 is connected to the projecting rod 664 on the underside of the plate surface 660 of the supporting plate 66 and the other end thereof is fixed to the inclined stud 693 of the holding plate 69 such that the pins 665 of the supporting plate 66 are engaged with slots of the fixing seat 692 to make the supporting plate 66 inclined toward the water collecting box 65. The parallel rails 712 of the bottom plate 71 are used to support the heating element 70 which the grooves 711 thereof are used to secure the holding plate 69. The bottom plate 71 is fixed to the fixing pieces 605 of the case 60 by screws (a) to enclose the supporting plate 66 and the holding plate 69 in the interior of the case 60. Then, the scattering plate 61 is engaged with the slots 603 of the case 60. Finally, the upper cover 64 is pivotally connected to the case 60 by engaging the flanges 640 with the corresponding ears 600.

In addition, a cleaned chopping board is placed on the plate surface 660 of the supporting plate 66 whereby the protuberances 660a on the plate surface 660 may form a space between the chopping board and the plate surface 660 such that the water coming from the wet chopping board may drop onto the plate surface 660 and then flow into the water collecting box 65 disposed at the outlet of the supporting plate 66. Meanwhile, the weight of the chopping board forces the supporting plate 66 downward to contact the microswitch 67 which in turn actuates the sterilizing lamp 62 and the heating element 70 to generate ultraviolet light and heat respectively. The scattering plate 61 is used to scatter the ultraviolet light generated by the sterilizing lamp and the heat produced by the heating element 70 to the whole interior of the case 60. The finished container can be attached to any desired location by means of conventional adhesive means because it has a compact volume resulting from the simplified elements and the reduced menber of same.

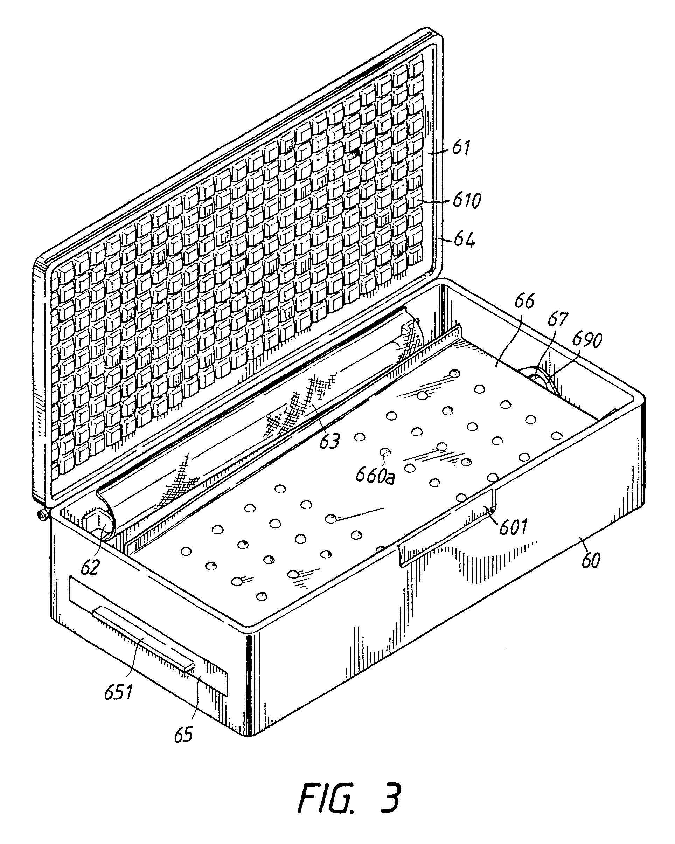

Referring to FIG. 3, a second preferred embodiment of the present invention comprises a case 60 in which a sterilizing lamp 62, a screen cover 63, a water collecting box 65, a supporting plate 66, a compression spring 68, a holding plate 69, a heating element 70 and a bottom plate 71 are received. The above-identified components are substantially similar to those as shown in FIG. 1 and FIG. 2 except that the dimensions of them have been modified in accordance with the case 60. It is to be noted that a scattering plate 61 is disposed in an upper cover 64 which is hinged to the longitudinal edge of the case 60. In operation, the chopping board is placed on the supporting plate 66 such that the weight of the chopping board forces the supporting plate 66 downward to contact the microswitch 67 in order to actuate the sterilizing lamp 62 and the heating element 70 whereby the ultraviolet light generated by the sterilizing lamp together with the heat produced by the heating element 70 are uniformly distributed by the scattering plate 61 to achieve both sterilizing and drying efficacy.

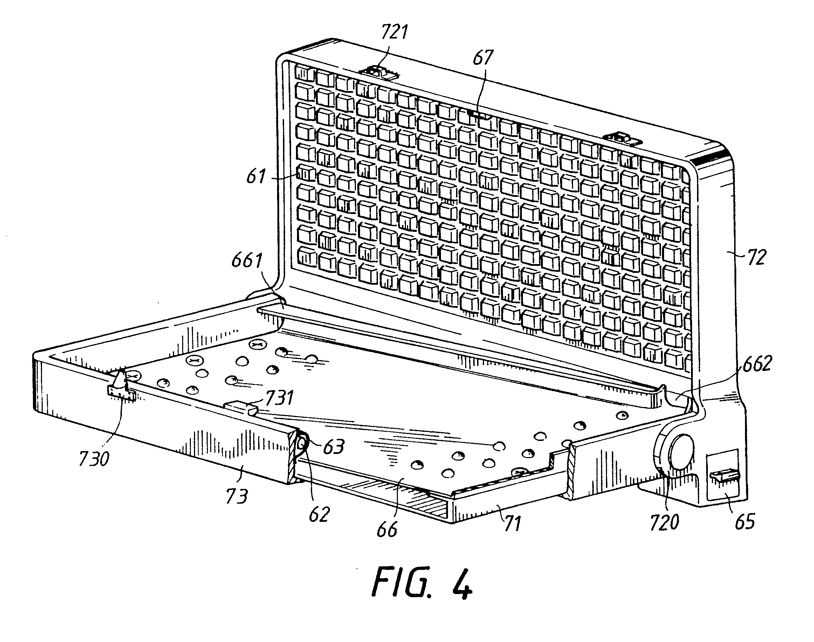

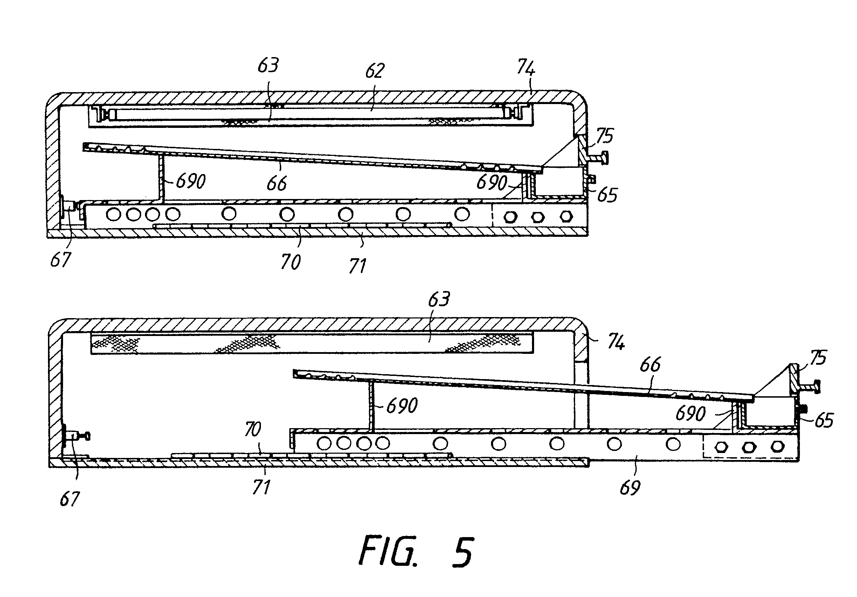

Referring to FIG. 4, a third preferred embodiment of the present invention mainly comprises a fixed case 72 and a moveable case 73 pivotally connected to the fixed case 72. A bottom plate 71, a heating element 70, a supporting plate 66, a sterilizing lamp 62 and a screen cover 63 are all disposed in the moveable case 73 wherein the supporting plate 66 together with the bottom plate 71 are secured to the moveable case 73. The supporting plate 66 has a plurality of protuberances 660a on the surface thereof and is provided with a slot 661 which is arranged to be inclined toward a water collecting box 65 and with an outlet 662. The heating element 70 is received in the bottom plate 71. In assembly, the fixed case 72 may be properly mounted to the wall of the kitchen and then the moveable case 73 is hinged to the fixed case 72. In operation, firstly, the cleaned chopping board is placed on the supporting plate 66 and then the fixed case 72 is covered with the moveable case 73 by inserting each male fastener 730 of the moveable case 73 into the corresponding female fastener 721 of the fixed case 73 in order to enclose the chopping board therein. Then, the chopping board slides into the slot 661 of the supporting plate 66 and is inclined toward the outlet 662 such that the water coming from the wet chopping board may flow into the water collecting box 65 via the slot 661. Meanwhile, a projecting block 731 of the moveable case 73 contacts the microswitch 67 formed in the inner wall of the fixed case 72 to actuate the sterilizing lamp 62 and the heating element 70 for achieving both sterilizing and drying effect when the male fasteners 730 are engaged with the female fasteners 721. Referring to FIG. 5, a fourth preferred embodiment of the present invention mainly comprises a drawer type case 74, a supporting plate 66, a holding plate 69, a microswitch 67, a water collecting box 65, a knob 75, a sterilizing lamp 62, a screen cover 63, a heating element 70, a scattering plate 61 and a bottom plate 71. The sterilizing lamp 62, the screen cover 63 and the scattering plate 61 are properly disposed in the case 74 while the bottom plate 71 are secured to the bottom of the case 74. The heating element 70 is properly located on the surface of the bottom plate 71. The holding plate 69 is slidably disposed in the case 74. The holding plate 69 is provided at the front end with a knob 75 and at the upper surface with a pair of supporting members 690 whereby the outlet of the supporting plate 66 is arranged to correspond to the water collecting box 65.

In operation, firstly, the holding plate 69 is drawn out of the case 74. Then, the holding plate 69 is pushed into the case 60 after the cleaned chopping board has been placed on the supporting plate 66. After the holding plate 69 is received in the case 74, the rear end of the holding plate 69 contacts the microswitch 67 formed on the inner wall of the case 74 to actuate the sterilizing lamp 62 and the heating element 70 whereby the ultraviolet light generated by the sterilizing lamp 62 and the heat produced by the heating element 70 can be uniformly distributed within the interior of the case 74 to achieve both sterilizing and drying effect.

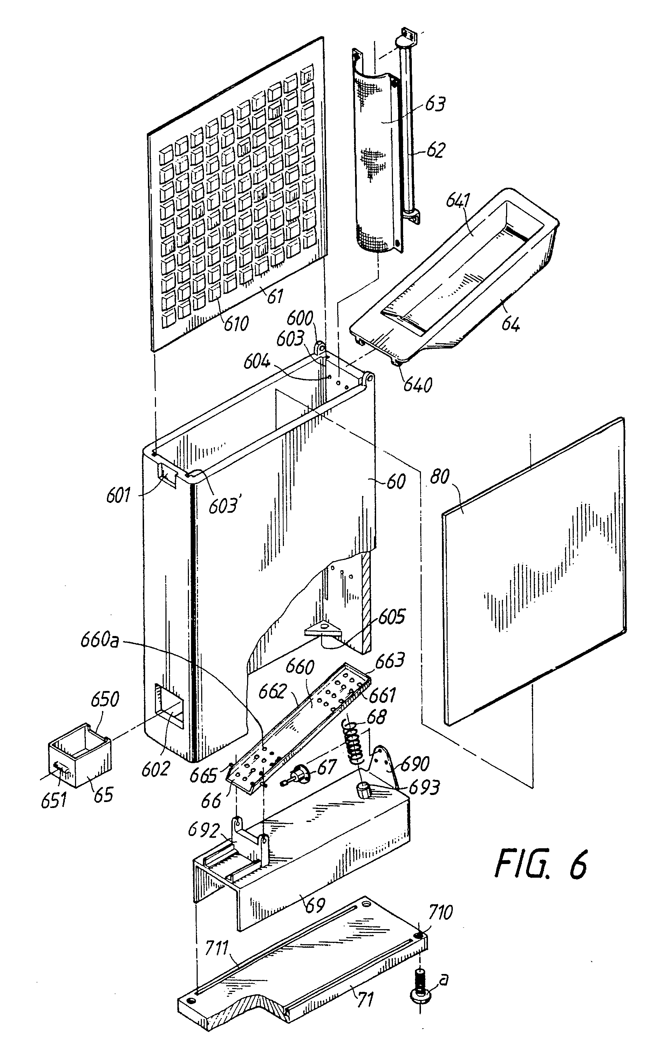

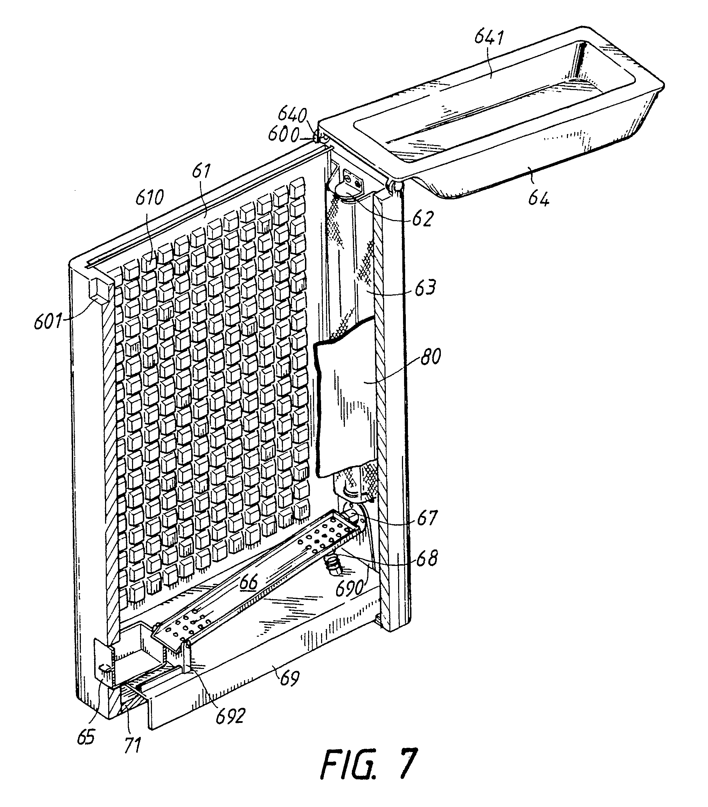

Referring to FIG. 6 and 7, a square plate 80 made of ceramic material is used to substitute for the conventional heating element. The case 60 is provided at its inner wall with a pair of grooves 603 to locate the ceramic plate 80 which is opposite to the scatering plate 61. Alternatively, the ceramic material may be directly located on the inner wall of the case 60 to form a heating layer. As is commonly known, the ceramic material can be processed to have semiconductor characteristics by utilizing the process of doping or nonstoichiometry. These ceramic materials having such semiconductor characteristics can meet requirements for various applications. For instance, a doped SiC can be pocessed to become a high temperature, stable semiconductor material to serve as a resistive heating element. In this way, the temperature up to at least $100^{\circ}$ C can be reached under any kinds of operating conditions by merely controlling the resistively as well as the cross sectional area of SiC.

The features disclosed in the foregoing description, in the claims and/or in the accompanying drawings may, both, separately and in any combination thereof, be material for realising the invention in diverse forms thereof.

## Claims

1. A container for storing and sterilizing a chopping board comprising:
A rectangular case being provided at the upper end with a pair of ears and a recess, a lonitudinal inner wall of said case having a predetermined amount of grooves and threaded holes, each of the four lower corners of said case being furnished with a connecting piece, the other longitudinal wall of said case being formed with an opening which corresponds to a water collecting box;

A cover being jointed to the open end of said case to cover said case;
At least one scattering plate, which is made by punching a flat reflective plate to form a plurality of geometry protuberances, being engaged with said grooves in said case;
At least one sterilizing lamp associated with a screen cover being disposed at the inner wall of the case;
An inverted U-shaped holding plate having a plurality of apertures, said holding plate being provided with a fixing seat, an inclined stud, and a connection member, one end of a compression spring being engaged with said inclined stud, a microswitch being secured to said connection member;
An U-shaped supporting plate having a plurality of protuberances, one end of said supporting plate being hinged to said fixing seat of said holding plate, the other end of saids upporting plate being contacted with said compression spring such that said supporting plate is inclined toward said water collecting box;
A bottom plate being secured to said four connecting pieces of said case, said bottom plate being provided with a pair of supporting rails which are used to support a heating element and with a pair of grooves by which said holding plate is located;
Whereby the ultraviolet light generated by said sterilizing lamp together with the heat produced by said heating element can be scattered by said scattering plate to enhance both sterilizing and drying effect.

2. A container as claimed in claim 1, wherein said scattering plate is disposed in the inner wall of said cover.

3. A container as claimed in claim 1, wherein said case is provided at the center of one horizontal inner wall with a microswitch and at the outer wall thereof with a pair of female fasteners, said case further being provided at the lower longitudinal wall with a slot to receive said water collecting box and with a pair of ears, said cover being hinged to said ears of said case, said cover being provided at the horizontal wall with a projecting block corresponding to said microswitch and with a pair of male fasteners corresponding to said female fasteners of said case whereby said projecting block contacts said microswitch when said case is enclosed with said cover by inserting said male fasteners into said female fasteners, said supporting plate disposed in said cover being formed with a substantially U-shaped slot which is inclined toward said water collecting box, and with a water outlet corresponding to said water collecting box.

4. A container as claimed in claim 1, wherein said microswitch is disposed at the rear wall of said case, said bottom plate being provided with a pair of sliding slots by which said holding plate is slidably received in said case, said holing plate being furnished at the front end with an inverted U-shaped knob and with an opening below said knob to receive said water collecting box, whereby the rear end of said holding plate contacts said microswitch disposed at the rear wall of said case when said holding plate is received into said case.

5. A container as claimed in claim 1, wherein said heating element is a square plate of ceramic material.

6. A container as claimed in claim 1, wherein a ceramic material is located on the inner wall of said case to serve as a heating element.

7. A container for storing and sterilizing a chopping board, comprising a case for receiving and enclosing a cleaned chopping board, the case containing heating means, sterilizing lamp means and scattering means and being provided with switch means operable when the chopping board is received in the case to actuate the heating means and the sterilizing lamp means to produce heat and ultraviolet light which are distributed and scattered by the scattering means to provide effective heating and sterilization at the chopping board.

FIG. 1

FIG. 2

FIG. 3

FIG. 4

EP 0 411 187 A1

FIG. 5

FIG. 6

*FIG. 7*

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | EP-A-0 105 814 (H. KUSAKARI) * Claims; figures * | 1-7 | A 61 L 2/10 A 47 J 47/00 |
| A | US-A-3 571 939 (B. PAUL) | | |

TECHNICAL FIELDS
SEARCHED (Int. Cl.5)

A 61 L

The present search report has been drawn up for all claims

| Place of search | Date of completion of the search | Examiner |
|---|---|---|
| THE HAGUE | 27-04-1990 | COUSINS-VAN STEEN G.I.L. |